# EUROPEAN PATENT SPECIFICATION

(11) **EP 2 235 542 B1**
(45) Date of publication and mention of the grant of the patent: **27.07.2016**
(21) Application number: 08865667.3
(22) Date of filing: 19.12.2008
(51) Int. Cl.: G01N 33/86

(54) **DIAGNOSTIC IN VITRO METHOD FOR ASSESSING VON WILLEBRAND DISEASE AND INCREASED BLEEDING RISK ASSOCIATED WITH VON WILLEBRAND DISEASE AND ACQUIRED OR CONGENITAL DISORDERS OF PLATELET FUNCTION**
DIAGNOSTISCHES IN-VITRO-VERFAHREN ZUR BEURTEILUNG DER VON-WILLEBRAND-KRANKHEIT UND MIT DER VON-WILLEBRAND-KRANKHEIT ASSOZIIERTE BLUTUNGSGEFAHR UND ERWORBENE ODER ANGEBORENE PLÄTTCHENSTÖRUNGEN
PROCÉDÉ IN VITRO DE DIAGNOSTIC POUR ÉVALUER LA MALADIE DE VON WILLEBRAND ET LE RISQUE SAIGNEMENT ASSOCIÉ AVEC LA MALADIE DE VON WILLEBRAND ET LES TROUBLES ACQUIS OU CONGÉNITAUX DE FONCTION PLAQUETTAIRE

(30) Priority: 21.12.2007 EP 07024932; 10.10.2008 EP 08105554
(43) Date of publication of application: 06.10.2010
(73) Proprietor: CSL Behring GmbH, 35041 Marburg (DE)
(72) Inventor: TOPF, Hans-Georg, 91054 Erlangen (DE); RAUH, Manfred, 96158 Frensdorf (DE)
(74) Representative: Hauser, Hans-Peter
(86) International application number: PCT/EP2008/010899
(87) International publication number: WO 2009/080303

(56) References cited:
- WO-A-97/46886
- WO-A-99/41615
- US-A- 5 951 951
- KAWASAKI ET AL.: "A-162: Effects of Platelet Agonists on Thromboelastogram in the Presence of Heparin or Argatroban. A-534: Do Platelet activators (Epinephrine, ADP and Collagen) Affect Whole Blood Clot Formation on Thromboelastogram?" ANNUAL MEETING OF THE AMERICAN SOCIETY OF ANESTHESIOLIGISTS, XX, XX, 1 January 2003 (2003-01-01), pages 1-4, XP002995736
- SWALLOW R A ET AL: "Thromboelastography: potential bedside tool to assess the effects of antiplatelet therapy?" PLATELETS (EDINBURGH), EDINBURGH, vol. 17, no. 6, 1 September 2006 (2006-09-01), pages 385-392, XP009100913 ISSN: 0953-7104
- CRAFT ROBERT M ET AL: "A novel modification of the thrombelastograph assay, isolating platelet function, correlates with optical platelet aggregation" JOURNAL OF LABORATORY AND CLINICAL MEDICINE, vol. 143, no. 5, May 2004 (2004-05), pages 301-309, XP002482807 ISSN: 0022-2143
- HARLE C C: "Point-of-care platelet function testing" SEMINARS IN CARDIOTHORACIC AND VASCULAR ANESTHESIA, WESTMINSTER, vol. 11, no. 4, 1 January 2007 (2007-01-01), pages 247-251, XP009100906 ISSN: 1089-2532
- FRESSINAUD E ET AL: "Screening for von Willebrand disease with a new analyzer using high shear stress: a study of 60 cases." BLOOD 15 FEB 1998, vol. 91, no. 4, 15 February 1998 (1998-02-15), pages 1325-1331, XP002483122 ISSN: 0006-4971
- FAVALORO EMMANUEL J: "Laboratory identification of von Willebrand disease: Technical and scientific perspectives" SEMINARS IN THROMBOSIS AND HEMOSTASIS, STUTTGART, DE, vol. 32, no. 5, 1 July 2006 (2006-07-01), pages 456-471, XP009100905 ISSN: 0094-6176

## Description

The invention relates to an in-vitro method for diagnosing Von Willebrand Disease (VWD) and an increased bleeding risk associated with von Willebrand Disease and/or acquired or congenital platelet function defects which reduce the interaction of von Willebrand Factor (VWF) with platelets. The in-vitro method may also be used to diagnose further bleeding risks. The test is suitable for use as a screening test based on whole blood and has the additional benefit of being suitable as a point of care test.

VWF is a multimeric adhesive glycoprotein present in the plasma of mammals, which has multiple physiological functions. During primary hemostasis VWF acts as a mediator between specific receptors on the platelet surface and components of the extracellular matrix such as collagen. Moreover, VWF serves as a carrier and stabilizing protein for procoagulant FVIII. VWF is synthesized in endothelial cells and megakaryocytes as a 2813 amino acid precursor molecule. The precursor polypeptide, pre-pro-VWF, consists of a 22-residue signal peptide, a 741- residue pro-peptide and the 2050-residue polypeptide found in mature plasma VWF (Fischer et al., FEBS Lett. 351: 345-348, 1994). Upon secretion into plasma by endothelial cells and by megakaryocytes VWF circulates in the form of various species with different molecular sizes. These VWF molecules consist of oligo- and multimers of the mature subunit of 2050 amino acid residues. VWF can be usually found in plasma as one dimer up to multimers consisting of 50 - 100 dimers (Ruggeri et al. Thromb. Haemost. 82: 576-584, 1999). The in vivo half-life of human VWF in the human circulation is approximately 12 to 20 hours.

VWF is known to stabilize FVIII in vivo and thus plays a crucial role to regulate plasma levels of FVIII and as a consequence is a central factor to control primary and secondary hemostasis. It is also known that after intravenous administration of pharmaceutical preparations containing VWF to von Willebrand disease (VWD) patients, an increase in endogenous FVIII:C to 1 to 3 units per ml in 24 hours can be observed demonstrating the in vivo stabilizing effect of VWF on FVIII.

VWF can be prepared from human plasma for example as described in EP05503991. EP0784632 describes a method for isolating recombinant VWF.

The most frequent inherited bleeding disorder in humans with a prevalence of about 0.8% to 1.3% is von Willebrand syndrome or von Willebrand disease (VWD), which can be treated by replacement therapy with concentrates containing VWF of plasmatic or recombinant origin.

There are four hereditary types of VWD described - type 1, type 2, type 3, and platelet-type. There are inherited and acquired forms of VWD. Most cases are hereditary, but acquired forms of VWD have been described. The International Society on Thrombosis and Haemostasis's (ISTH) classification depends on the definition of qualitative and quantitative defects.

### Type 1 VWD

Type 1 VWD (60-80% of all VWD cases) is a quantitative defect but may not have clearly impaired clotting. Most patients usually end up leading a nearly normal life. Complications may arise in the form of bleeding following surgery (including dental procedures), noticeable easy bruising, or menorrhagia (heavy periods). Decreased levels of VWF are detected (10-45% of normal, i.e. 10-45 IU).

### Type 2 VWD

Type 2 VWD (10-20%) is a qualitative defect and the bleeding tendency can vary between individuals. There are normal levels of VWF, but the multimers are structurally abnormal, or subgroups of large or small multimers are absent. Four main subtypes exist: 2A, 2B, 2M and 2N.

### Type 2A VW D

This is an abnormality of the synthesis or proteolysis of the VWF multimers resulting in the presence of small multimer units in circulation. Factor VIII binding is normal. Type 2A VWD causes a disproportionately low ristocetin cofactor activity compared to the von Willebrand's antigen.

### Type 2B VWD

This is a "gain of function" defect leading to spontaneous binding to platelets and subsequent rapid clearance of the platelets and the large VWF multimers. A mild thrombocytopenia may occur. The large VWF multimers are absent in the circulation and the Factor VIII binding is normal.

### Type 2M VWD

This is caused by decreased or absent binding to GPIb on the platelets. Factor VIII binding is normal.

### Type 2N (Normandy) VWD

This is a deficiency of the binding of VWF to factor VIII. This type gives a normal VWF antigen level and normal functional test results but has a low Factor VIII. This has probably led to some 2N patients being misdiagnosed in the past as having hemophilia A.

### Type 3 VWD

Type 3 (1-3%) is the most severe form of VWD where no functional VWF is present and may cause severe mucosal bleeding, no detectable VWF antigen, and may also cause sufficiently low Factor VIII levels that hemarthoses (joint bleeding), as in cases of mild hemophilia occur.

### Platelet-type VWD

Platelet-type VWD is an autosomal dominant type of VWD caused by gain of function mutations of the VWF receptor on platelets; specifically, the alpha chain of the glycoprotein Ib receptor (GPIb). This protein is part of the larger complex (GPIb/V/IX) which forms the full VWF receptor on platelets. The ristocetin activity and loss of large VWF multimers is similar to type 2B, but genetic testing of VWF will reveal no mutations.

Besides quantitative or qualitative defects of VWF the absence of specific receptors on the platelet surface or other platelet abnormalities can affect VWF dependent platelet aggregation. The VWF molecule has specific binding sites for the platelet receptors GPIb and GPIIb/IIIa, FVIIIC, collagen, sulfatides, heparin, and the snake venom derived protein botrocetin. Platelet aggregation is resulting from inter-platelet contacts. This contact is mediated by fibrinogen and VWF and requires the presence of receptors on the platelet surface. In their resting state platelets are non-thrombogenic, but expose receptors when platelet aggregation is initiated and platelets are activated. Lack of corresponding receptors for VWF or impaired activation cause severe bleeding, as seen for the Bernard Soulier syndrome, a congenital disorder with a deficiency of GPIb. In contrast to the "gain of function" mutation of GPIb in platelet-type VWD, in Bernard-Soulier syndrome a "loss of function" mutation in the VWF receptor GPIb causes the clinical phenotype. The clinical phenotype is comparable to VWD but neither the amount nor the activity of VWF is changed.

### Acquired von Willebrand disease

Acquired VWD is a bleeding disorder with laboratory findings similar to those for congenital VWD. Unlike the congenital form, acquired VWD usually occurs in individuals with no personal or family history of bleeding disorders. Acquired VWD is associated with lymphomyeloproliferative, immunological and cardiovascular disorders, as well as with solid tumors and other miscellaneous conditions. Acquired vWD can occur in patients with autoantibodies. In this case the function of vWF is not inhibited but the VWF-antibody complex is rapidly cleared from the circulation. Acquired VWD is probably the cause of many cases of acquired bleeding disorders which are not diagnosed because specific tests for VWD are missing in most of routine laboratories. Another form of acquired VWD occurs in patients with aortic valve stenosis, leading to gastrointestinal bleeding (Heyde's syndrome).

Presently, a number of tests, many of which are complex and time consuming, are required for the diagnosis and classification of VWD, as no single test is ideal. Some are general coagulation tests assessing a general bleeding risk like the determination of PTT, FVIII:C and bleeding time, whereas other tests are related to VWF specifically like the determination of VWF antigen (VWF:Ag).

Currently available routine screening tests in coagulation, for example platelet count, the activated partial thromboplastin time, and bleeding time, are insensitive to the detection of coagulation abnormalities caused by VWD or platelet defects which reduce the interaction between VWF and platelets. The bleeding time is quite variable and does not correlate well with plasma VWF antigen (VWF:Ag) or ristocetin cofactor activity (VWF:RCo). The bleeding time, therefore, is not a useful screening test for VWD.

More elaborate VWF tests are VWF antigen (WF:Ag), ristocetin cofactor activity (VWF:RCo), the collagen binding assay (VWF:CBA), analysis of ristocetin induced platelet aggregation (RIPA), the VWF:FVIII binding assay and the especially time consuming analysis of the VWF multimer pattern. VWF protein is quantified by enzyme-linked immunosorbent assay (ELISA) procedure or newer automated technologies. It does not reflect active, functional, or adhesive properties of VWF. Currently, the ristocetin cofactor assay is the only routinely used functional assay of VWF which is performed using platelet agglutination. However, it has a number of limitations including poor reproducibility and poor inter-laboratory correlation. The VWF:CBA has been developed as an alternative functional assay, based on ELISA technique which has the potential to be more reproducible. The VWF:CBA is particular sensitive to loss of high molecular-weight VWF multimers. VWF:FVIII binding assay assesses the ability of VWF to bind FVIII and is used to diagnose Type 2N.The RIPA procedure requires an individual's platelet rich plasma for sensitivity to ristocetin at various concentrations. Ristocetin sensitivity is dependent on both the level and function of VWF. VWF:multimer assay involves gel electrophoresis detecting VWF of differing molecular weight, and identifying certain VWF structural abnormalities. Multimeric analysis will allow the allocation to the different subtypes. Because of test complexity, time and cost, it is only performed by a small number of expert laboratories. Also due to the time delay and sample handling when sending samples to expert laboratories, sometimes erroneous results are obtained due to loss of high molecular weight VWF forms.

In addition VWD diagnosis and platelet dysfunction can also be performed with the automated analyzer PFA100 (Siemens Medical Solution Diagnostics) which measures primary hemostasis under high shear conditions by aspirating anticoagulated blood samples at a constant negative pressure through a small aperture. A platelet plug forms at the aperture and the time required for the cessation of blood flow (closure time) to occur is determined. Platelet function and VWF levels have been shown to be important determinants of the closure time (E.J. Favaloro, Seminars in Thrombosis and hemostasis 32: 456-471, 2006). In assays based on the PFA100 neither the intrinsic nor the extrinsic plasmatic coagulation cascade is induced, e.g. plasmatic coagulation resulting in the formation of fibrin does not take place.

US 5,951,951 proposes an improved method to measure activated clotting time in citrated blood samples containing platelets by introducing a pre-test-recalcification step in which an anticoagulant ensures that clotting does not start prematurely. It is proposed to add to the citrated blood sample simultaneously (a) a platelet function restoration agent, (b) an anticoagulant and (c) to at least one part of the sample a platelet activating agent. After the pre-test-recalcification clotting is induced by addition of a clotting agent. Clotting can then be measured in various ways including the determination of a change in viscosity. The function of the platelet activating agent is that it enhances in normal conditions the ability of platelets to effectively participate in the blood clotting reaction and thereby shortens the clotting time. Thus the addition of the platelet activating agent leads in samples from healthy, normal donors to an increase in viscosity as compared to a sample from the same donor to which no platelet activator is added, whereas in samples from donors with platelet defects the addition of the platelet activating agent does lead to a reduced shortening of the clotting time, e.g. a reduced increase in viscosity or to no change at all, as compared to a sample from the same donor to which no platelet activator is added. The test method disclosed has the object of providing a method for detecting platelet defects.

WO 99/41615 discloses a method to assess the coagulant activity of blood by measuring the rate of blood clotting in the presence of certain metal ions. A sample from a donor is split into at least two samples to at least one of which metal ions are added. In one embodiment of the invention a change in viscosity is measured. In this embodiment plasmatic coagulation is not induced but rather a metal ion is added to at least one sample and the relative change of viscosity between the two samples is subsequently measured. Before measuring the change in viscosity optionally a platelet activator may be added. However this method does not teach adding a platelet activator only to one sample and not to the other sample and to compare the difference in the amount of viscoelastic change caused by this differential treatment.

In recent years the analytical technique of thrombelastography was introduced into clinical practice. Thrombelastography (TEG) is a diagnostic method which mechanically investigates clot formation or dissolution in an oscillating system. Here, either a vessel (cup) is in oscillating motion around a measuring rod (pin) (conventional TEG) or else the vessel is fixed and the pin is brought into oscillating rotational motion (ROTEG or ROTEM). The mechanical forces arising between the cup and pin are recorded. As soon as the blood or plasma starts to clot, a variation in the initial measurement signal occurs. Both designs will be designated here as TEG.

TEG is employed for the investigation of blood or plasma in order to determine clotting-relevant parameters (TEG parameters), such as the period of time up to reaching a first significant clot formation with an amplitude of 2 mm (clotting or reaction time r), the period of time up to reaching a clot thickness of an amplitude of 20 mm (k value), the rate at which the clot is formed (alpha angle), the mechanical properties of the clot at maximum amplitude (MA) or at any other desired point in time, the period of time up to MA (TMA), or the period of time until the clot strength has fallen again to a certain value because of fibrinolysis. Clinically, TEG is employed as a diagnostic measure, inter alia, for the assessment of coagulopathy, for example in heart surgery, in liver transplantation, major abdominal surgery and as a quasi-bedside test in perioperative clotting management.

TEG is available in addition to the standard clotting diagnostics (thromboplastin time, aPTT, platelet count, AT III, fibrinogen, D dimers, bleeding time), which are more time-consuming for the determination of the values for rapid information about bleeding trends. This is particularly of importance if coagulopathies occur in the course of extensive surgical interventions or after polytraumata, since serious haemostasis disorders can rapidly lead to the development of secondary tissue damage and are often resistant to a conventional haemostatic therapy based on a more time-consuming standard clotting diagnosis.

The attraction of the method lies in its global aspect, the output being influenced by coagulation factors and inhibitors, platelets, and fibrinolytic pathways (Luddington, Clin. Lab. Haem. (2005) 27, 81-90). This gives the potential to identify the principal mechanisms contributing to defective hemostasis and coagulation within the sample in a more meaningful way than the much more artificial coagulation screening tests. The TEG parameters are influenced by a number of factors especially fibrinogen, factor XIII, and blood platelet levels and components of the fibrinolytic system such as plasmin, plasmin activators, and plasmin inhibitors. For instance, fibrinogen, as a clotting substrate, correlates with the clot stability. This can be clearly shown in the thrombelastogram. Also the absence of coagulation factors like FVIII or FIX can be detected by thromboelastography.

The clot is formed by interaction of fibrin with activated platelets. Fibrin strands and platelet aggregates determine both the mechanical strength of the formed clot and the viscoelastic properties of the sample. Aggregated platelets associated with VWF and fibrin contribute to about 80% and fibrin contributes to about 20% of the viscoelastic properties (e.g. the maximal amplitude as measured in TEG) in normal samples.

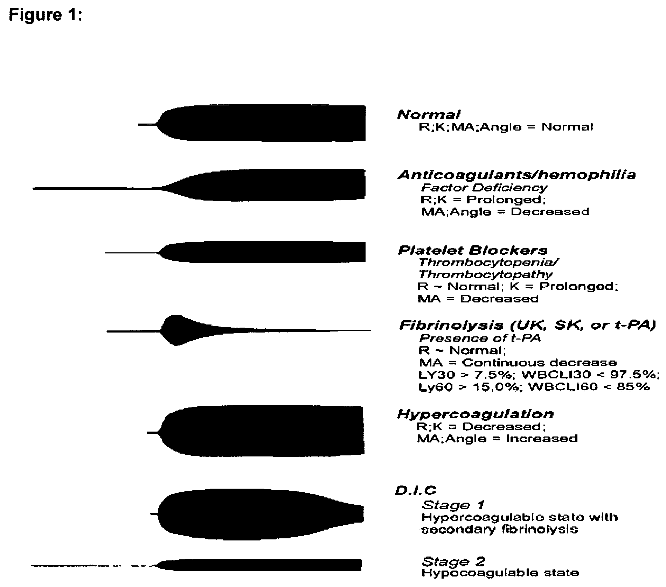

As discussed above thrombelastography is currently used for the analysis of several defects in coagulation, like hemophilia A and B, thrombocytopenia, thrombopathy, hypercoagulation as well as defects in the fibrinolysis system (see figure 1) (R.J. Luddington, Clin. Lab. Haem. 27: 81-90, 2005). WO 2006/084648 teaches using TEG for differentiating a Factor XIII from a fibrinogen deficiency state.

Recently, a modified rotation thrombelastogram (ROTEM®, Pentapharm, Munich, Germany) has avoided several limitations of classical TEG. Four channels are available for simultaneous measurements. Each test requires 300 µl citrated whole blood. Activation of coagulation can be performed by different activators making it possible to evaluate different activation pathways. Four standard ROTEM® assays, named INTEM, EXTEM, NATEM, and FIBTEM, can be performed according to protocols supplied by the manufacturer. The INTEM and EXTEM represent assays in which the intrinsic or the extrinsic coagulation pathways are triggered. NATEM (Non-Activated TEM) is used to assess whole blood clot formation in the absence of activation of the clotting cascade other than recalcification and spontaneous contact activation. Finally, the FIBTEM assay can be used for the assessment of the specific role of fibrinogen in clot formation following inhibition of the platelets by Cytochalasin D. As for INTEM and EXTEM, 300 µL of citrated blood are recalcified with 20 µL of CaCl2 0.2 M (star-TEM® reagent, Pentapharm GmbH, Munich, Germany) and the coagulative cascade is activated by partial thromboplastin phospholipids made of rabbit brain plus ellagic acid (in-TEM® reagent, Pentapharm GmbH, Munich, Germany) and thromboplastin from rabbit brain (ex-TEM® reagent, Pentapharm GmbH, Munich, Germany), respectively. As for FIBTEM, 300 µL of citrated blood is mixed to 20 µL of ex-TEM® reagent plus 20 µL of cytochalasin D/DMSO solution, 0.2 M CaCl2 (fib-TEM® reagent, Pentapharm GmbH, Munich, Germany).

ROTEM® has been shown to be a point of care device for rapid diagnosis in various bleeding disorders and for global assessment of coagulation status in patients with hypercoagulable conditions. In particular this method has the potential to illustrate hypocoagulation typically found in disorders such as haemophilia, miscellaneous coagulation factor deficiencies and platelet disorders. Furthermore, experimental ex vivo substitution with various haemostasis-promoting components have revealed that ROTEM® is useful to illustrate the correction of a compromised haemostatic capacity.

However, TEG has not been used so far for the diagnosis of an increased bleeding risk associated with VWD or platelet defects which reduce the interaction of VWF with platelets.

In clinical practice it is necessary to determine the bleeding risk of a patient before undergoing surgery. When a general coagulation test like e.g. the PTT is increased indicating an increased bleeding risk, in order to determine the cause for the increased PTT several further tests need to be performed involving also often some more time consuming test as the e.g. the multimer analysis of VWF.

If results from these tests are not yet available, patients who do not suffer from life-threatening indications are not allowed to undergo the scheduled surgical operation and surgery is postponed often up to several weeks.

Therefore there is a high medical need for a test to identify the bleeding risk of an individual patient as well as his VWF function which test should provide rapid results, ideally being a point of care test suitable for whole blood analysis requiring only small blood samples. The problem solved by the present invention was to provide such a test.

Surprisingly it has been found in the present invention that there is a way to use the determination of a change in viscoelasticity in thromboelastography in assessing deficiencies of VWF including both qualitative as well as quantitative deficiencies of VWD and bleeding risk associated with von Willebrand Disease and acquired or congenital platelet function defects which reduce the interaction of VWF with platelets. The present invention provides a rapid VWD and platelet function defect assay including a bed-side or point of care test suitable for the use of whole blood based on thromboelastography.

Even more surprisingly it was found that the test is able to determine patients suffering from platelet storage pool diseases and from dysfibrinogenemia. Platelet interaction seems to require a multistep process, and different mechanisms through different receptors seem to be involved. Interactions between various platelet adhesive proteins such as fibrinogen or von VWF and their respective surface receptors mediates platelet interaction and may result in a clinically manifest bleeding diathesis. This is also reported for secreted intracellular proteins from platelet granules during platelet activation.

It has also been found that the test can be performed with body fluids, preferably blood, in volumes as small as 105 µl per test which is a huge advantage especially in diagnosing paediatric patients and newborns. Therefore the invention encompasses also tests to determine the bleeding risk of a given patient due to VWD, platelet defects, platelet storage pool diseases and dysfibrinogenemia in blood samples of at most 300µl per test, preferably at most 200 µl per test and most preferably at most 105 µl per test. A most preferred embodiment of the invention is determining the bleeding risk from blood samples smaller than 105 µl.

### Figures:

**Figure 1****:**
   Figure 1 shows a normal thrombelastogram compared to thrombelastograms of various defects in coagulation
**Figure 2****:**

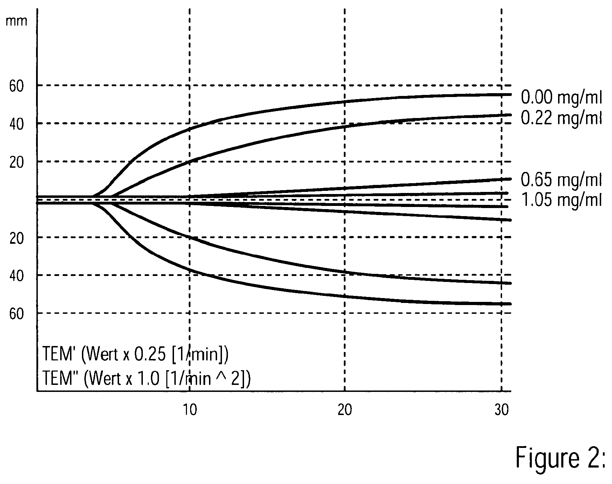

   Figure 2 shows that by incubating citrated blood with increasing amounts of ristocetin (up to a final concentration of 1.05 mg/ml) the maximum amplitude is decreased and the clotting time is increased.
**Figure 3****:**

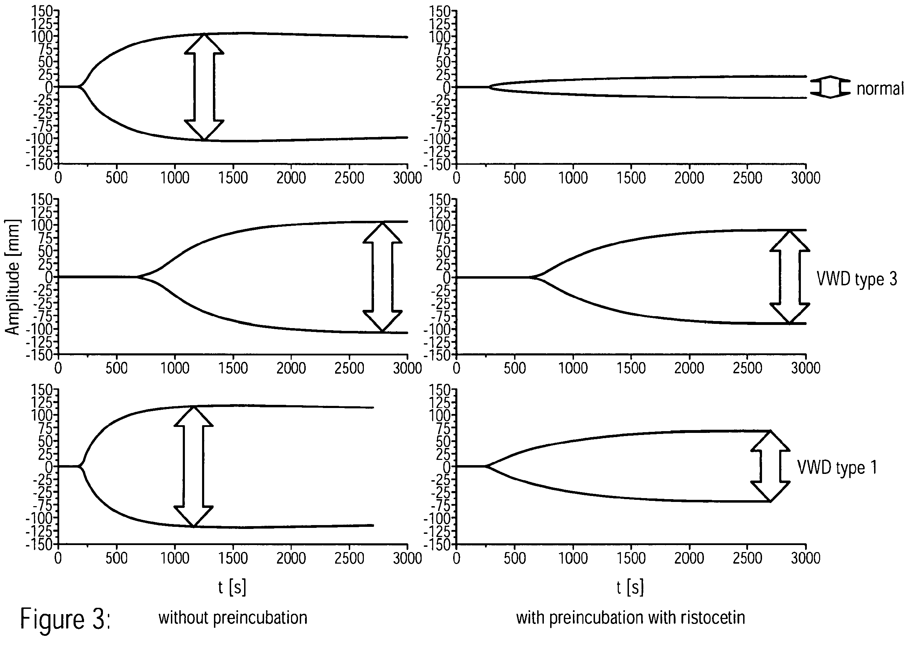

   Figure 3 shows that when a thromboelastographic analysis of normal blood is performed the maximal amplitude after a preincubation with ristocetin reaches only about 20% of the maximal amplitude without such preincubation with ristocetin. In blood from a VWD type 3 patient a maximal amplitude of almost 100% is reached even with a ristocetin preincubation, and in blood from a VWD type 1 patient is about 75%.
**Figure 4****:**

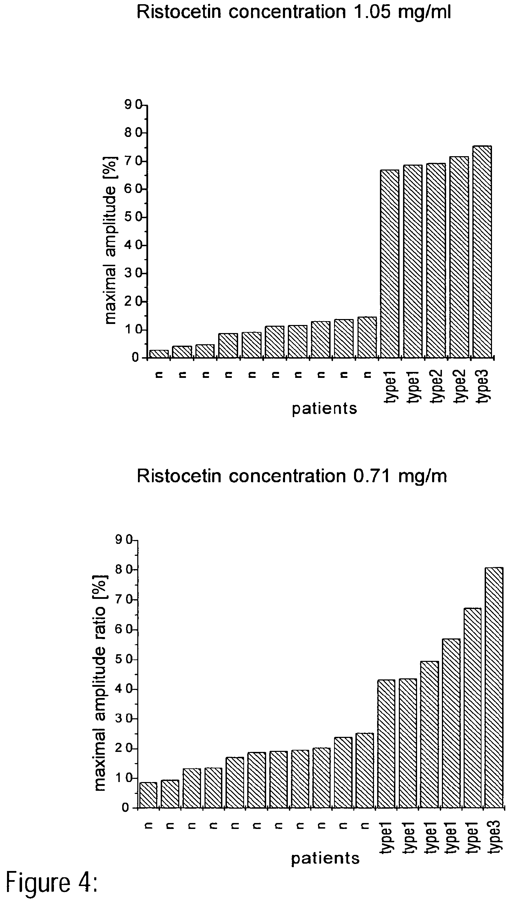

   Figure 4 shows that the ratio of the maximal amplitude with a preincubation with ristocetin over the maximal amplitude without a preincubation with ristocetin provides a diagnostic parameter to differentiate between healthy individuals and patients having VWD. The decrease of the maximal amplitude is a function of the ristocetin concentration as seen in figure 3 and figure 2.
**Figure 5****:**

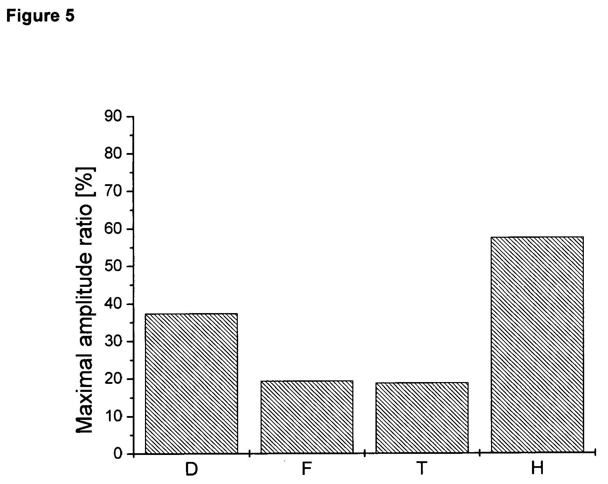

   Figure 5 shows the ratio of the maximal amplitude for members of a family who all suffer from VWD Type 1. The patients exhibit a wide variation in their clinical manifestation which correlates to the amplitude ratio observed.
**Figure 6****:**

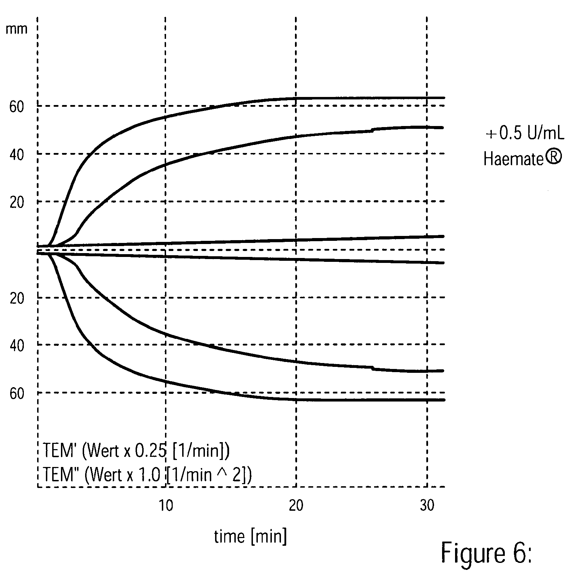

   Figure 6 shows the normalization of the ratio of the maximal amplitude ([(maximal amplitude in a blood sample from a donor with added ristocetin) / (maximal amplitude in a blood sample without added ristocetin)] *100) after adding Haemate (0.5 U/ml) in vitro to the citrated blood of a VWD type 3 patient.
**Figure 7****:**

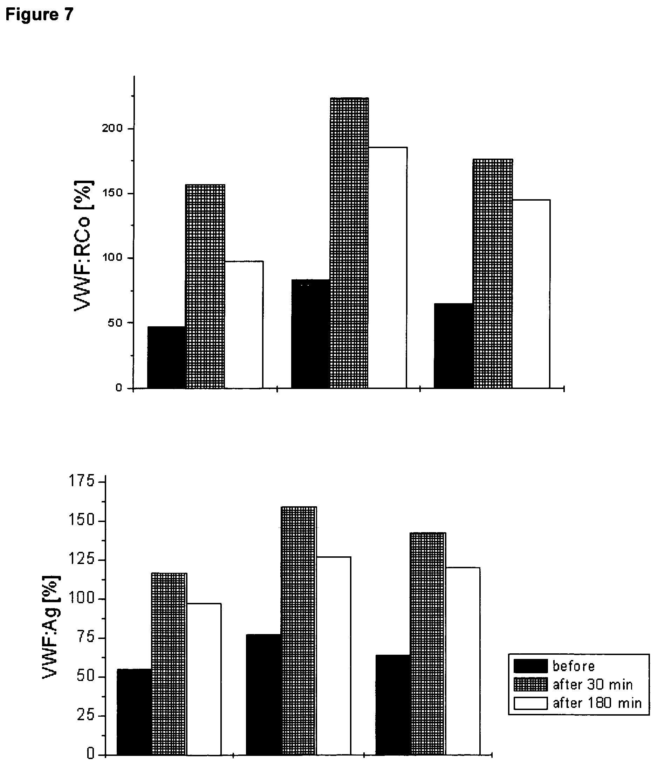

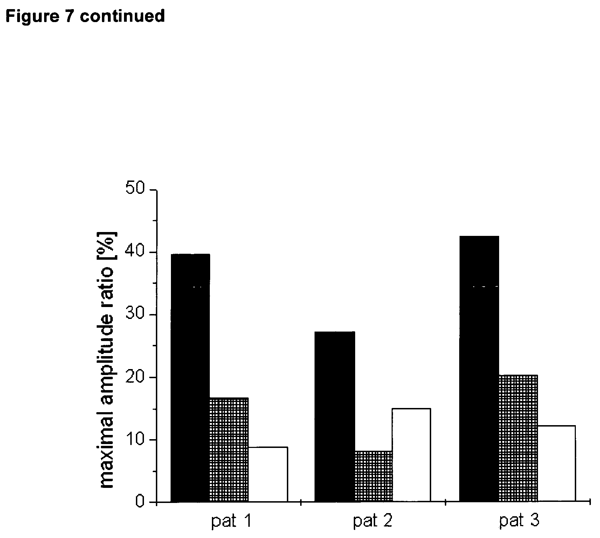

   Figure 7 shows the increase of ristocetin cofactor activity and of VWF:Ag and the corresponding decrease of the ratio of the maximal amplitude ([(maximal amplitude in a blood sample from a donor with added ristocetin) / (maximal amplitude in a blood sample without added ristocetin)] *100) after administration of DDAVP in three VWD type 1 patients (pat 1 to pat 3) at three time points (before, 30 min after and 180 min after administration of DDAVP).
**Figure 8****:**

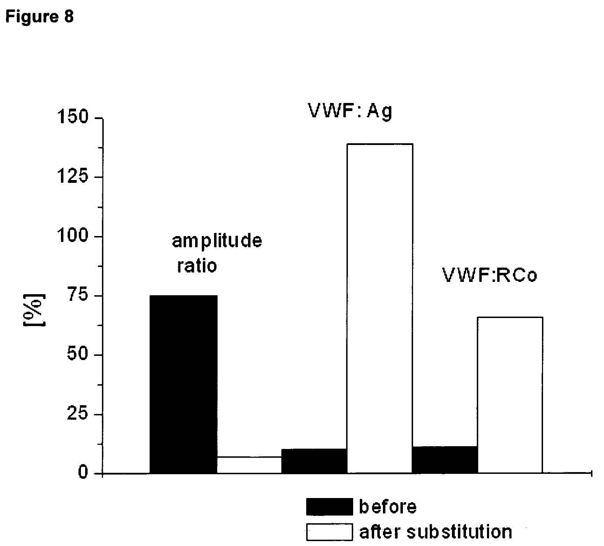

   Figure 8 shows the ratio of the maximal amplitude ([(maximal amplitude in a blood sample from a donor with added ristocetin) / (maximal amplitude in a blood sample without added ristocetin)] *100), the ristocetin cofactor activity and VWF-Ag before and after administration of VWF concentrate in a VWD type 2 patient before and after substitution with a VWF concentrate (Haemate® 33 U/kg).
**Figure 9****:**

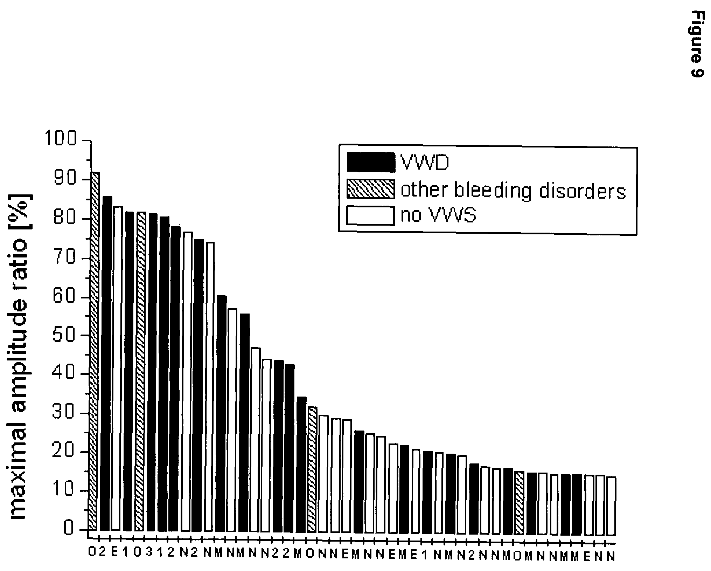

   Figure 9 shows samples wherein the ratio of the maximal amplitude was determined to be more than or equal to 15% ([(maximal amplitude in a blood sample from a donor with added ristocetin) / (maximal amplitude in a blood sample without added ristocetin)] *100 ≥ 15%). Patients were categorized into three groups according the classification of the reference laboratory. (group 1: patient with VWD **(1:** VWD type 1, **2:** VWD type 2, **3:** VWD type 3, **M:** mild phenotype VWD type1, group 2: **O:** patients with other classified bleeding disorders, group 3: patients without VWD, **N:** no VWD, **E:** equivocal VWD))
**Figure 10****:**

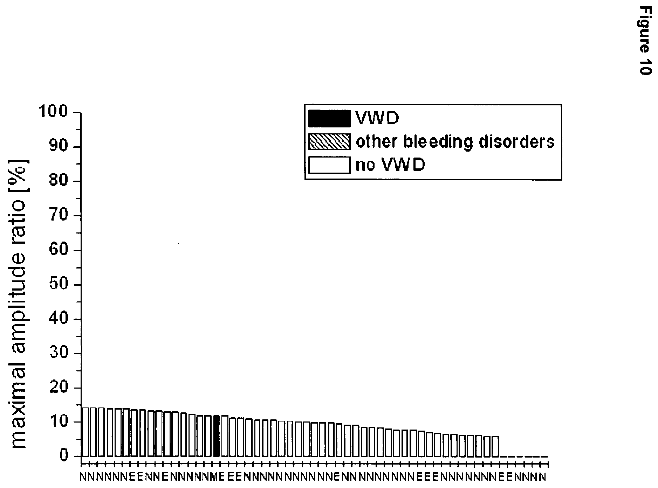

   Figure 10 shows samples wherein the ratio of the maximal amplitude was determined to be less than 15% ([(maximal amplitude in a blood sample from a donor with added ristocetin) / (maximal amplitude in a blood sample without added ristocetin)] *100 < 15%).. Patient were categorized into three groups according the classification of the reference laboratory. (group 1: patient with VWD **(1:** VWS type 1, **2:** VWD type 2, **3:** VWD type 3, **M:** mild phenotype typ1, group 2: **O:** patients with other classified bleeding disorders, group 3: patients without VWD, **N:** no VWD, **E:** equivocal VWD))

### Summary of the Invention

The invention relates to an in-vitro method for diagnosing von Willebrand Disease and bleeding risk associated with von Willebrand Disease and platelet function disorders. The method is suitable for use as a screening method based on whole blood and has the additional benefit of being suitable as a point of care diagnostic method. The method of the invention is superior in predicting bleeding risk as compared to known diagnostic methods like platelet count, the activated partial thromboplastin time, the prothrombin time and bleeding time.

### Detailed Description of the Invention

The invention relates to the measurement of viscoelastic changes and to comparing the amount of viscoelastic changes in blood samples after coagulation is induced. The total amount of the viscoelastic change in a sample from a healthy person after coagulation is induced stems partly from the fibrin meshwork which is formed after plasmatic coagulation is induced and partly from the aggregation of activated platelets (which are activated during coagulation by thrombin), VWF and the fibrin meshwork.

The gist of the invention is that by incubating a blood sample from a healthy donor comprising platelets and VWF with an activator of platelet aggregation before plasmatic coagulation provides enough thrombin and fibrin to entangle the platelets in the sample will cause an almost complete preaggregation of platelets. This preaggregation has however as a prerequisite that the interaction between VWF and platelets is normal, meaning that the donor has no increased bleeding risk.

Because the preaggregated platelets do not contribute to the total amount of the viscoelastic change in a similar way as non-preaggregated platelets, if then in the sample in which platelets have preaggregated coagulation is induced and generates the fibrin meshwork only a reduced amount of viscoelastic change is found as compared to the amount of the viscoelastic change in a sample which had not been incubated with an activator of platelet aggregation. This lower amount of viscoelastic change in the sample which has been incubated with an activator of platelet aggregation will then be compared to either a reference value or to the amount of the viscoelastic change in a similar sample from the same donor which has not been incubated with an activator of platelet aggregation. A large difference between both amounts of viscoelastic changes translates into the diagnosis that the donor has no enhanced bleeding risk.

If on the contrary in a sample from a donor with an increased bleeding risk due to a compromised interaction between VWF and platelets, also the preaggregation of platelets and VWF is compromised and takes place to a lesser degree as compared to a sample from a healthy donor or not at all. When then coagulation is initiated now (since pre-aggregation took not place or to a lesser degree) the platelets can still contribute to the total amount of the viscoelastic change. Therefore in a sample from a donor with an increased bleeding risk a normal, large amount of viscoelastic change is measured after coagulation is induced also in a sample which was incubated with an activator of platelet aggregation. This normal, large amount of viscoelastic change in the sample which has been incubated with an activator of platelet aggregation will then be compared to either a reference value or to the amount of the viscoelastic change in a similar sample from the same donor which has not been incubated with an activator of platelet aggregation. The small difference between both amounts of viscoelastic changes translates into the diagnosis that the donor has an enhanced bleeding risk.

The reference values can be determined by way of non-limiting example in healthy donors without increased bleeding risk, by determining the amount of viscoelastic change after inducing coagulation either i) by incubating with an activator of platelet aggregation (=REFH+) ii) by not incubating with an activator of platelet aggregation (=REFH-).

Reference values can for example also be determined by using donor samples from donors with an increased bleeding risk.

Depending on how the reference value was determined the interpretation will be different as shown in Table 1 for reference samples generated from donors with no increased bleeding risk (REFH+ or REFH-).

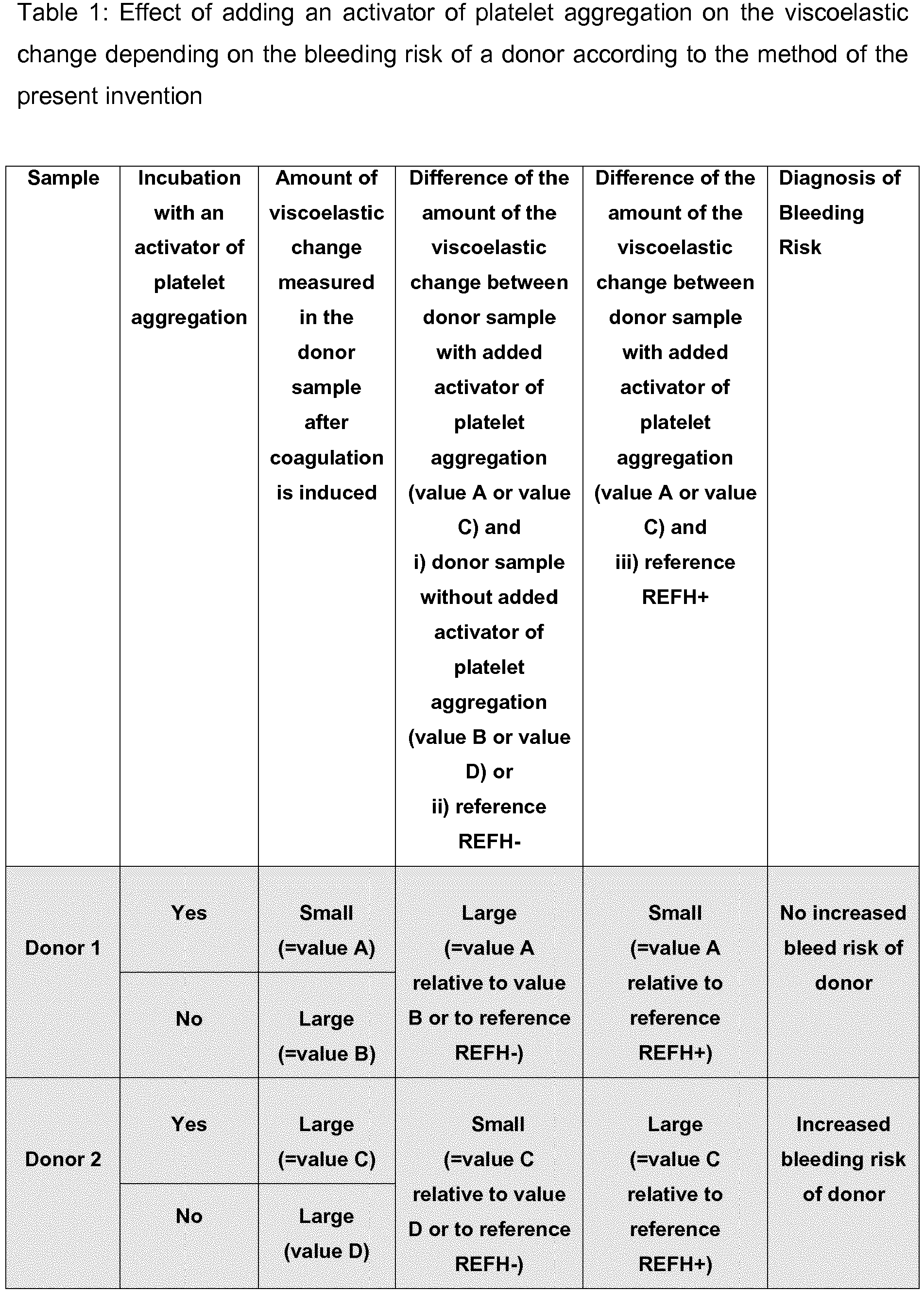

Therefore one aspect of the invention is a diagnostic in-vitro method to determine qualitative and/or quantitative defects of von Willebrand factor characterised by,
a) using a sample obtained from a human individual or an animal comprising a body fluid which comprises platelets and hemostasis factors,
b) incubating at least one part of the sample with an activator of platelet aggregation, wherein the activator of platelet aggregation is a compound which is able to induce VWF dependent platelet activation, aggregation and agglutination in a fluid derived from the human body which contains platelets and VWF by causing binding of VWF to a VWF-receptor on platelets,
c) inducing coagulation in the part of the sample which was incubated with an activator of platelet aggregation,
d) measuring the viscoelastic change occurring after induction of coagulation in the part of the sample which was incubated with an activator of platelet aggregation in a thromboelastograph, and
e) comparing the amount of the viscoelastic change determined in step (d) with a reference value,
   wherein the reference value is obtained:
   i) by splitting the original donor sample obtained in step (a) into at least two samples or obtaining in step (a) at least two samples from a donor wherein in at least one sample only step (a) and steps (c) to (e) are performed and step (b) is omitted and wherein the amount of viscoelastic change measured in the sample in which step (b) of the method of claim1 was omitted is the reference value; or
   ii) by applying a method comprising step (a) and steps (c) to (e) to a sample from a healthy donor or to several samples from healthy donors without increased bleeding risk wherein the amount of viscoelastic change measured in the sample or in the samples in which step (b) was omitted is the reference value; or
   iii) by applying a method comprising steps (a) to (e) to a sample from a healthy donor or to several samples from healthy donors without increased bleeding risk wherein the amount of viscoelastic change measured in the sample or in the samples is the reference value.

Thus in a sample from a VWF type 1 patient the amount of the viscoelastic change as determined in step (d) is lower compared to the amount of viscoelastic change which is measured if the same method is applied to another sample obtained in the same way as in step (a) from the same VWF type 1 patient but no activator of platelet aggregation is added.

A large difference between i) the amount of the viscoelastic change measured in a sample which has been incubated with an activator of platelet aggregation and ii) the amount of the viscoelastic change of a reference sample which has not been incubated with an activator of platelet aggregation results in the diagnosis that the donor of the sample has no enhanced bleeding risk;
whereas a reduced difference between i) the amount of the viscoelastic change measured in the sample which has been incubated with an activator of platelet aggregation and ii) the amount of the viscoelastic change of the reference sample which has not been incubated with an activator of platelet aggregation results in the diagnosis that the donor has an increased risk of bleeding.

A small difference between i) the amount of the viscoelastic change measured in a sample which has been incubated with an activator of platelet aggregation and ii) the amount of the viscoelastic change of the reference sample which has also been incubated with an activator of platelet aggregation results in the diagnosis that the donor of the sample has no enhanced bleeding risk;
whereas a large difference between i) the amount of the viscoelastic change measured in the sample which has been incubated with an activator of platelet aggregation and ii) the amount of the viscoelastic change of the reference sample which has also been incubated with an activator of platelet aggregation results in the diagnosis that the donor has an increased risk of bleeding.

Preferably step (b) of the above method is performed before step (c) of the above method.

Optionally an activator of coagulation may be added when performing the method of the invention. Preferably the activator of coagulation is added before or after the addition of an activator of platelet aggregation in step b) but before inducing coagulation in step c).

Unlike the method disclosed in US 5,951,951 the method of the invention does not require the addition of an anticoagulant. Therefore in a preferred embodiment of the invention no anticoagulant is added to the sample.

The original body fluid may be diluted and its composition might be changed as long as it still comprises platelets and haemostatic factors.

Preferably a ratio between the amount of viscoelastic change of the sample which has been incubated with an activator of platelet aggregation and the reference value as detailed above is determined in step e) of the method.

Multiple parameters which can be determined by TEG and which assess viscoelastic changes can be used for performing the method of the invention, such as determining the maximal amplitude (MA), the area under the curve, the rate at which the clot is formed (alpha angle), the amplitude at defined time points, derivation parameters, Preferred is determining the maximal amplitude or area in step e) of the method of the invention in both parts of the original sample and more preferred is determining the ratio of the maximal amplitude or the area under the curve of both parts of the sample in step (f).

"Viscoelastic change" in the sense of the invention is any change in viscosity and shear-elasticity of the sample during the coagulation process and formation of the clot.

"Amount of viscoelastic change" in the sense of the invention is the difference between the viscosity of the sample before inducing coagulation and after a certain point in time after coagulation has been induced. The preferred point in time to measure the amount of viscoelastic change may vary and depends also on the parameter which is actually measured.

"Activator of platelet aggregation" in the sense of the invention is any composition of matter or any compound which is able to induce VWF dependent platelet activation, aggregation and agglutination in a fluid derived from the human body which contains platelets and VWF by causing binding of VWF to a VWF-receptor on platelets. Preferred activators of platelet aggregation are ristocetin and botrocetin. A most preferred activator of platelet aggregation is ristocetin.

"Incubating" with an activator of platelet aggregation in the sense of the invention is to add the activator of platelet aggregation to a sample comprising platelets and VWF for at least 0,1 min, preferably for at least 1 min, most preferably for at least 5 min.

"Hemostasis factors" in the sense of the invention is a group of coagulation factors including components of the intrinsic and the extrinsic coagulation cascade, thrombin and fibrinogen which do generate fibrin from fibrinogen once coagulation is activated.

"Inducing coagulation" in the sense of the invention means any way of triggering or activating the coagulation system depending on the type of sample and anticoagulant used comprising recalcification, addition of antagonists of added anticoagulants, or any other substances leading to the generation of fibrin from fibrinogen. In whole untreated blood coagulation may already be induced by contact to surfaces like for example the surface of the cuvette where the blood is incubated. If citrated blood or platelet rich plasma is used coagulation can be induced by the addition of calcium. In case of heparinized blood or platelet rich plasma, coagulation can be induced by the addition of heparinase.

Preferably an activator of platelet aggregation is added prior to inducing aggregation. However the invention can also be practiced by simultaneous induction of coagulation and addition of an activator of platelet aggregation. Less preferred but still a workable embodiment of the invention comprises adding the activator of platelet aggregation after coagulation has been induced.

"Activators of coagulation" in the sense of the invention are substances that activate one or more coagulation factors, but which on their own do not lead to the generation of fibrin in the respective fluid comprising a body fluid comprising platelets and haemostasis factors. A preincubation with such activators before actually inducing coagulation offers significant advantages such as shortening of reaction times by controlled activation versus surface contact activation only and improved precision. A preferred activator of coagulation is kaolin. Moreover, it gives the possibility for additional differential diagnostic information.

Preferably an activator of coagulation is added before inducing coagulation.

If functional VWF is present in the sample and platelet function is normal the addition of an activator of platelet aggregation prior, during or after inducing the coagulation process leads to platelet aggregation and platelet activation. These pre-aggregated platelets show a different interaction with the fibrin which is formed from fibrinogen in the clot formation once coagulation is induced. This is leading to a much reduced change in viscoelasticity in the sample as compared to a sample in which no activator of platelet aggregation was added. In such samples from healthy individuals one does measure for example a large MA in a TEG without an added activator of platelet aggregation, but a small MA if an activator of platelet aggregation has been added.

In contrast if VWF with quantitative of qualitative defects is present in the sample, less or no platelets aggregate once an activator of platelet aggregation is added. Therefore when coagulation is induced, more or all platelets are still available which have not yet aggregated and which can still interact with the fibrin which is formed during the coagulation process. The qualitative or quantitative defect of VWF can therefore be determined for example by a large MA in a TEG if an activator of platelet aggregation has been added.

The method of the present invention is also able to differentiate between different types of VWD. For example as shown in Figure 3, when a thromboelastographic analysis of normal blood is performed the maximal amplitude after a preincubation with ristocetin reaches only about 20% of the maximal amplitude without such preincubation with ristocetin. In blood from a VWD type 3 patient a maximal amplitude of almost 100% is reached even with a ristocetin preincubation, and in blood from a VWD type 1 patient the maximal amplitude is reduced to about 75%.

The addition of activators of platelet aggregation and the subsequent binding of activator-VWF complexes to platelets leads to the aggregation of platelets and also to their activation. This activation depends on the binding of VWF complexes to corresponding platelet receptors. So, the response to said activators of platelet aggregation is also affected by lack of corresponding platelet receptors or impaired platelet function. Therefore for example a large MA in a TEG after treating the sample with an activator of platelet aggregation can also be caused by defects in the platelets leading to an impaired VWF dependent platelet aggregation, like e.g. in case of the Bernard Soulier Syndrome.

If a large change in viscoelasticity like a large MA in a TEG is determined by practising the method of the invention a diagnosis of qualitative or quantitative defects of VWF or impaired VWF dependent platelet aggregation can be based a) on the comparison of the large change in viscoelasticity with a reference value based on predetermined change of viscoelasticity in samples of healthy individuals or b) by comparison to a reference part of the original sample which has not been incubated with an activator of platelet aggregation and in which coagulation has been also induced.

Therefore the method of the invention offers a rapid way to assess the absence or function impairment of VWF and VWF factor related platelet dysfunction and such offers a rapid method to diagnose VWD and platelet function disorders which reduce the interaction of VWF with platelets. Even more surprisingly it was found that the test is able to determine patients suffering from platelet storage pool diseases and from dysfibrinogenemia. The method of the invention is suitable for bed-side testing obviating the need of other much more time consuming diagnostic methods. Another advantage of the method of the invention is that it eliminates the need for time-consuming centrifugation steps. The test can be performed with blood volumes as small as 105 µl which is a huge advantage especially in diagnosing paediatric patients and newborns.

Such a test is especially useful when a bleeding risk due to VWF impairment or platelet function disorders like Bernard Soulier syndrome needs to be determined when results are needed rapidly like for example in the pre- and perioperative setting. Several disorders and comorbidites as well as several drugs are known to impair platelet function.

As the change in viscoelasticity as exemplified in a thrombelastogram reflects the global status of the coagulatory and the fibrinolytic system in a given donor, also congenital deficiencies in certain coagulation factors like in hemophilia A or hemophilia B, depletion of coagulation factors like fibrinogen as in trauma or in surgical bleeding or pharmacological intervention like coumarin treatment will be reflected in certain characteristic patterns of the change in viscoelasticity or the thromboelastogram (Luddington, Clin. Lab. Haem. (2005) 27, 81-90) which the skilled artisan is able to interpret. Thus the use of the method according to the present invention not only enables the diagnosis of an increased bleeding risk due deficient VWF or platelet receptors which reduce the interaction of VWF with platelets but also enables the concomitant diagnosis of other abnormalities of the coagulatory or the fibrinolytic system.

Thus performing the method according to the present invention also allows diagnosing hyperfibrinolysis, multifactorial coagulopathy due to blood loss, bleeding due to the use of anticoagulants, trauma associated hypocoagulatory state, pregnancy associated hypercoagulatory state, disseminated intravascular coagulation, thrombocytopenia, congenital bleeding disorders leading to the lack of functional coagulation factors like FVIII, FIX, FXIII and fibrinogen, platelet storage diseases and dysfibrinogenemia.
The use of reagents and kits suitable for assessing von Willebrand disease and bleeding risk associated with von Willebrand Disease and acquired or congenital platelet function defects by determining viscoelastic changes is also part of the present invention.

Most surprisingly it was found that even in cases when the conventional VWF diagnosis is not able to diagnose a differential bleeding risk between different patients the method of the invention as shown in Example 4 (a) predicts the bleeding propensity of a given patient in a more predictive way than known methods. As shown in Example 4 (b) individuals falsely diagnosed to have VWD (probably due to loss of VWD multimers during storage and transport which is a common phenomenon) were correctly diagnosed with the in-vitro diagnostic method of the invention to be healthy and having no increased bleeding risk. The method of the invention therefore is in at least some cases more reliable and robust than conventional VWD diagnostic methods.

It could also be shown (Example 5) that the method of the invention can be used to monitor success of failure of different treatment regimens like the administration of DDAVP to VWD or hemophilia A patients or the administration of VWF concentrates to VWD or hemophilia A patients.

Perhaps one of the most intriguing features of the method of the invention is that it not only offers a sensitive and specific test method to diagnose VWD and/or platelet defects which affect the binding of VWF to platelets but in addition identifies patients with an increased bleeding risk, which risk is sometimes due to additional causes with sometimes unclear pathogenesis.

This might be due to the fact that the method of the invention determines the bleeding risk of an individual in full blood, where plasmatic and cellular components of the coagulation system are present and are not artificially separated as is the case in many other coagulation test system.
The method of the invention is therefore a fast method to reliably predict a bleeding risk in a given individual and also narrows down possible causes for such bleeding risk.

The method of the invention is preferentially performed as described in the examples.

A fluid comprising platelets and haemostasis factors as whole blood or anticoagulated blood (for example citrated blood or citrated platelet rich plasma or heparinized blood or heparinized platelet rich plasma) from a patient is transferred to a vial optionally containing a standardized agent which acts as an activator of coagulation e.g. kaolin, or tissue factor but without actually inducing coagulation at this point in time.

Preferably the components are mixed by inversion and optionally divided into at least two aliquots. An amount of an activator of platelet aggregation is added to one of the aliquots. If ristocetin is used the final concentration is preferably between 0.01 and 100 mg/ml, more preferable between 0.1 and 3mg/ml or even more preferably between 0.3 mg/ml and 1.5 mg/ml.

The aliquots are preferably incubated for 0 to 360 min at 10 to 37 °C, more preferably 5 min at about room temperature, or 10 min at about 37°C preferably while being mixed.

Then both aliquots are transferred to a container which allows the determination of the viscocelastic change, e.g. TEG assay cups.

Preferably then coagulation is induced, but coagulation may also be induced simultaneously or less preferred before the addition of an activator of platelet aggregation.

The viscoelastic change is measured in both aliquots, if TEG is used to determine the viscoelastic change, the assay is preferentially run until the maximal amplitude is reached.

The optional sample without addition of an activator of platelet aggregation like ristocetin is used as a control measurement.

If TEG is the assay to determine the viscoelastic change the platelet activation in response to the activator of platelet aggregation for example ristocetin can by way of non limiting example be determined from the maximum amplitudes in the control without added platelet activator (MA) and the sample with the added platelet activator Ristocetin (MA_{Ristocetin}): which can then be expressed as the ratio (MA_{Ristocetin}/MA) *100. The percentage change is determined to assess individual subject's responses, thus providing an indication of relative response to a certain dose of ristocetin. It was found that at a ristocetin concentration of 0.75 mg/ml a ratio of 15% is a preferable cut-off to determine patients which do have an increased bleeding risk.
If the ratio (MA_{Ristocetin}/MA) *100 is larger than or equal to 15% patients have an increased bleeding risk and suffer either from VWD or other bleeding diathesis and should not undergo surgery before further diagnosis if no life-threatening condition requires immediate surgery.
If the ratio (MA_{Ristocetin}/MA) *100 is lower than 15% there is no enhanced bleeding risk and patients can undergo surgery.

It has to be understood that this ratio of 15% is not limiting and for example only. Depending on the technique to determine the viscoelastic change, depending on the individual parameter measured the man skilled in the art can determine a ratio based on samples for example from VWD patients and from healthy patients which differentiates pathological states from healthy states.

An object of the invention is a diagnostic in vitro method wherein a value of (viscoelastic change in a sample of a body fluid from a donor with added activator of platelet aggregation/(viscoelastic change in a sample of the same body fluid without added activator of platelet activation) of more than or equal to 0.15 is interpreted to be an indicator of a bleeding risk. The bleeding risk may be due to VWD but may have other causes as well as outlined above.

Patients without VWD show a significant decrease of the maximum amplitude after incubation with ristocetin as described in example 2. Almost all platelets are deactivated and no more platelet contribution to the clot strength after the subsequent induction of coagulation is detectable. In contrast, for patients with VWD the clot strength is not or only slightly modified by the ristocetin incubation.

In summary the in-vitro diagnostic method of the invention is especially suitable as a screening test. A positive result in this initial screening test will then be in most cases followed up by further specific laboratory test like VWF multimer determination or platelet function tests etc.

### Examples:

### Example 1:

Citrate-stabilized blood from a healthy patient was transferred to a vial containing a standardized kaolin reagent and mixed by inversion The kaolin reagent was purchased from Haemoscope Corporation. The mixture was mixed well and divided into two 500 µL aliquots. Different amounts of ristocetin at a concentration of 15 mg mL⁻¹ was added to one part of the activated blood aliquots and both samples were incubated on a mixer for 10 min. 300 µL of both aliquots were filled to TEG assay cups and 20 µL of 0.2 M CaCl₂ were added. The TEG tracings were allowed to run as least until the maximal amplitude was reached using a Rotem Analyser. Figure 2 shows four distinct TEG patterns obtained. Dependent of the final ristocetin concentration a decrease of the maximal amplitude and an increase in the clotting time were observed.

### Example 2:

Analyser and the reagents were from Haemoscope Corporation. 1.3 mL of blood was collected in 3.18% trisodium citrate (1:9 anticoagulant to blood). One millilitre of citrate-stabilized blood was transferred to a vial containing a standardized kaolin reagent and mixed by inversion. The mixture was mixed well and divided into two 500 µL aliquots. 25 µL ristocetin at a concentration of 15 mg mL⁻¹ was added to one part of the activated blood aliquots and both samples were incubated on a mixer for 10 min. 360 µL of both aliquots were filled to TEG assay cups and 20 µL of 0.2 M CaCl₂ were added. The TEG tracings were allowed to run as least until the maximal amplitude was reached. Figure 3 shows three distinct TEG patterns obtained. Fig 1 a shows the TEG profiles of a normal healthy person and figure 1 b and 1 c shows the TEG profiles of patients with VWD type 3 and type 1.

### Example 3:

According to the procedure in example 2 blood samples from healthy patients and patients with from VWD were tested. All VWD samples derived from patients diagnosed as having VWD using standard criteria. In addition to VWD plasma, a number of normal samples were also collected and used for comparative studies. The platelet activation in response to ristocetin is determined from the maximum amplitudes in the control (MA) and ristocetin activated trace (MA_{Ristocetin}): MA_{Ricocetin}/MA*100. The percentage change was calculated to assess individual subject's responses, thus providing an indication of relative response to a certain dose of ristocetin (0.71 mg/ml and 1,05 mg/ml final concentration). The Kaolin induced sample without adding ristocetin acted as the control measurement. Patients without VWD show a significant decrease of the the maximum amplitude after incubation with ristocetin as described in example 2. Almost all platelets are deactivated and no more platelet contribution to the clot strength is detectable. In contrast, for patients with VWD the clot strength is not or only slightly modified by the ristocetin incubation.

### Example 4:

a) The diagnosis of VWD remains problematic, and this is particularly so for type 1 VWD. Many of these subjects do not exhibit bleeding manifestations, and thus go unrecognized. Studies of family members show that there is variable penetrance and expressivity of the disease. Variability of both bleeding manifestations and laboratory results between affected family members indicates variable penetrance and expressivity of VWD mutations (Miller CH, Graham JB, Goldin LR, Elston RC. Genetics of classic von Willebrand's disease. I. Phenotypic variation within families. Blood 1979: 54: 117-36.)
   We investigated four members of a family who were all affected with VWD Type 1. A detailed bleeding and family history was taken from each subject by personal interview. The patients show different thromboelastographic (TEG) patterns which correlate with their clinical presentations. Patient H who has the highest ratio shows meno-metorrhagia, post dental extraction hemorrhage and cutaneous bleeding symptoms. In contrast, for the three other patients no striking bleeding manifestations were reported so far.
   Therefore the diagnostic method of the invention was superior to predict the bleeding risk of family member H than the classical diagnostic assays. No correlation was found for the coagulation screening tests prothrombin time and activated thromboplastin time.
b) Many preanalytical variables cause substantial problems for the identification of VWD. In addition, VWF, and in particular high molecular weight VWF, is very sensitive to collection and transport artifacts. Low-temperature transport and/or storage of sodium citrate plasma will give rise to loss of HMW VWF in a subset of samples. In these cases, a misidentification or false conclusion of VWD may arise when laboratory test results are accepted without repeat confirmation.

Surprisingly in 103 samples tested we found that the in-vitro diagnostic method of the invention detected correctly the absence of VWD in two cases where a conventional diagnosis based on multimer determination lead to a misclassification of patients to have VWD. Both patients were classified according to conventional diagnosis based on the multimer pattern as type 2A whereas the in-vitro diagnostic method of the invention identified no hint for a bleeding risk/VWD as the TEG ratios were in the normal range. A second sample of the patients was retested then and showed now no abnormalities in the multimer pattern.

### Example 5:

### a.) In vitro therapy control (VWF concentrate)

Citrated blood of a patient with VWD Type 3 was incubated with different amounts of haemate, a commercial VWF concentrate for the replacement therapy. The mixture was mixed well and divided into two 500 µL aliquots. 25 µl of ristocetin at a concentration of 15 mg mL⁻¹ was added to one part of the activated blood aliquots and both samples were incubated on a mixer for 10 min. 300 µL of both aliquots were added to TEG assay cups of a ROTEM® analyser containing 20 µL star-TEM reagent for recalcification and 20 µl ex-TEM reagent. The extrinsic clotting cascade is triggered by the added tissue factor. A ROTEM® Analyser was used for recording the TEG tracings at least until the maximal amplitude was reached. As shown in figure 6 the ratio is normalized and is not significantly different from healthy patients after adding Haemate in vitro.

### b.) In vivo therapy control (DDAVP)

Therapy in VWD may be necessary to alleviate a bleeding episode or to prevent bleeding episode. Most individuals with type 1 VWD generally are managed effectively using DDAVP (Desmopressin acetate), which acts to release endogenous stored VWF. It is usual to conduct a trial of DDAVP and then monitor the response by subsequent blood sampling and testing. All patients had a high amplitude ratio before administration of DDAVP and DDAVP was able to normalize the TEG ratio ([(maximal amplitude in a blood sample from a donor with added ristocetin) / (maximal amplitude in a blood sample without added ristocetin)] *100). The method is a simple and rapid tool to discriminate responders from nonresponders to DDAVP. The advantage of this method is the close to immediate test time obtained. The result is available very soon after blood sampling. In contrast the VWF:CB result is generally obtained some days after blood sampling. The values for the amplitude ratio, the VWF:Ag and VWF-RCo are shown in figure 7 for three time points (before, 30 min after and 180 min after administration of DDAVP). This shows that also in vivo the diagnostic in-vitro method of the invention can be used to monitor the success of a therapeutic intervention with DDAVP to reduce a bleeding risk due to VWD.

### c.) In vivo therapy control (VWF concentrate)

Patients with moderate to severe factor VIII and von Willebrand factor deficiency usually require treatment. Providing normal VWF through the infusion of plasma derived concentrates can correct factor VIII and VWF deficiency. TEG assays with and without ristocetin preincubation were performed before and after administration. The effect of substitution of VWF could be seen in the significant decrease of the ratio of the maximal amplitudes in the TEG test ([(maximal amplitude in a blood sample from a donor with added ristocetin) / (maximal amplitude in a blood sample without added ristocetin)] *100) as shown in figure 8. Accordingly, we had an increase in baseline values of plasma VWF:Ag and VWF-ristocetin cofactor activity from 10% respectively 11 % to 139% and 66% for both variables. This shows that also in vivo the diagnostic in-vitro method of the invention can be used to monitor the success of a therapeutic intervention substituting VWF to reduce a bleeding risk due to VWD.

### Example 6:

We evaluated the performance of this assay and its usefulness for laboratory diagnosis of VWD. 103 patients (58 women, 45 men; median age 11.9 ± 11.2) were enrolled in the study. Patients with preexisting hemophilia disorders which can be detected by the INTEM assay were excluded from the study. VWD classification was performed by a reference laboratory based on VWF:Ag, vWF:CB and VWF multimer pattern. For the three subgroups, results for maximal amplitude ratios ([(maximal amplitude in a blood sample from a donor with added ristocetin) / (maximal amplitude in a blood sample without added ristocetin)] *100) are shown in figure 9 and 10.

| **Classification** | | **Number** |
|---|---|---|
| VWD | | **21** |
| | Type 3 | 1 |
| | Type 2A | 4 |
| | Type 2B | 2 |
| | Type 1 | 3 |
| | Type 1 mild phenotype | 11 |
| | Equivocal (no VWD but borderline) | 17 |
| | Normal (no VWD) | 61 |
| Other | Unclassified platelet function defect | 1 |
| | Abdominal bleeding | 1 |
| | Platelet storage pool disease | 1 |
| | Dysfibrinogenemia | 1 |
| Total | | 103 |

As depicted in figures 9 and 10 the outcome was as follows:
a) TEG ratio < 15%
   → 56 samples from normal healthy individuals (xyz of them with "equivocal" VWD diagnostic result)
   → 1 sample from a patient with vWD type 1 (mild phenotype)
b) TEG ratio ≥ 15%
   → 1 sample from a patient with VWD type 3
   → 4 samples from patients with VWD type 2A
   → 2 samples from patients with VWD type 2B
   → 3 samples from patients with VWD type 1
   → 10 samples from patients with VWD type 1 (mild phenotype)
   → 26 samples from individuals with no VWD

According to a cutoff value of 15% for the amplitude ratio the sensitivity for detecting VWD was 95% ([(number of true positives/(number of true positives + number of false negatives)]: 20/21 = 95,2 %), the specificity for VWD was 68% ([(number of true negatives/(number of true negatives + number of false positives)]: 56/82 = 68,3%).

However among the 26 individuals having a TEG ratio ≥ 15% but who were not classified as VWD by the reference laboratory almost half had a clinical history pointing to other uncharacterized bleeding disorders. So far the following bleeding disorders could be assigned to:
→ one patient has an unclassified platelet function defect with strong gastrointestinal bleeding,
→ one patient has strong gastrointestinal bleeding caused by a lympho-proliferative syndrome,
→ one patient has a platelet storage pool disease,
→ one patient has a dysfibrinogenemia,
→ five are classified as equivocal VWD
→ Two patients have repeated bleeding episodes, epistaxis and prolonged bleeding times
→ Three individual having a family history of severe bleeding episodes

So it can be assumed that the specificity and sensitivity for bleeding disorders is much higher.

With regard to the diagnosis of a bleeding diathesis the sensitivity seems to be at least 95% (as the one patient with VWD type yxz which was not detected might when further examined show to have no increased bleeding risk) and a potential specificity of 82% (56/68 = 82,3%). Also the real specificity might even higher if the 15 remaining "false positive" individuals should upon further examination turn out to have an increased bleeding risk.

The in-vitro diagnostic method of the invention is therefore suitable to detect not only bleeding risks associated with VWD or platelet diseases affecting the interaction of VWF with platelets but is a general test suitable to detect bleeding risks.

## Claims

1. Diagnostic in-vitro method to determine qualitative and/or quantitative defects of von Willebrand factor **characterised by**,
a) using a sample obtained from a human individual or an animal comprising a body fluid which comprises platelets and hemostasis factors,
b) incubating at least one part of the sample with an activator of platelet aggregation, wherein the activator of platelet aggregation is a compound which is able to induce VWF dependent platelet activation, aggregation and agglutination in a fluid derived from the human body which contains platelets and VWF by causing binding of VWF to a VWF-receptor on platelets,
c) inducing coagulation in the part of the sample which was incubated with an activator of platelet aggregation,
d) measuring the viscoelastic change occurring after induction of coagulation in the part of the sample which was incubated with an activator of platelet aggregation in a thromboelastograph, and
e) comparing the amount of the viscoelastic change determined in step (d) with a reference value,
wherein the reference value is obtained:
i) by splitting the original donor sample obtained in step (a) into at least two samples or obtaining in step (a) at least two samples from a donor wherein in at least one sample only step (a) and steps (c) to (e) are performed and step (b) is omitted and wherein the amount of viscoelastic change measured in the sample in which step (b) was omitted is the reference value; or
ii) by applying a method comprising step (a) and steps (c) to (e) to a sample from a healthy donor or to several samples from healthy donors without increased bleeding risk wherein the amount of viscoelastic change measured in the sample or in the samples in which step (b) was omitted is the reference value; or
iii) by applying a method comprising steps (a) to (e) to a sample from a healthy donor or to several samples from healthy donors without increased bleeding risk wherein the amount of viscoelastic change measured in the sample or in the samples is the reference value.

2. Diagnostic in-vitro method according to claim 1 wherein the body fluid is whole blood, anticoagulated blood or platelet rich plasma.

3. Diagnostic in-vitro method according to claims 1 or 2 wherein the reference value obtained in alternative (ii) or (iii) of claim 1 is an average value of the amounts of viscoelastic change as measured in several samples obtained from healthy donors.

4. Diagnostic in-vitro method according to claims 1 to 3 wherein an activator of coagulation is added.

5. Diagnostic in-vitro method according to claims 1 to 4 wherein an activator of coagulation is added prior to step (b) or after step (b) but before inducing coagulation in step (c).

6. Diagnostic in-vitro method according to claims 1 to 5 wherein the viscoelastic change is measured by determining any one of the parameters of thromboelastography.

7. Diagnostic in-vitro method according to claim 6 wherein the parameter of thromboelastography is the maximal amplitude.

8. Diagnostic in-vitro method according to claims 1 to 7 wherein the activator of platelet aggregation is selected from the group comprising ristocetin and botrocetin.

9. Diagnostic in-vitro method according to claim 8 wherein the activator of platelet aggregation is ristocetin.

10. Diagnostic in-vitro method according to claim 9 wherein the ristocetin concentration is between 0.1 mg/ml and 1.5 mg/ml.

11. Diagnostic in-vitro method according to claims 1 to 10 for predicting the bleeding risk of a patient prior to surgery or during therapy.

12. Diagnostic in-vitro method according to claims 1 to 11, wherein the sample of the body fluid is at most 105 µl per test.

13. Diagnostic in-vitro method according to the claims 1 to 12, wherein a value of (viscoelastic change in a sample of a body fluid from a donor with added activator of platelet aggregation)/(viscoelastic change in a sample of the same body fluid without added activator of platelet activation) of more than or equal to 0.15 is interpreted to be an indicator for a bleeding risk.

14. Use of the diagnostic in-vitro method according to claims 1 to 13 for therapy control wherein the therapy comprises the administration DDAVP.

15. Use of the diagnostic in-vitro method according to claims 1 to 13 for therapy control wherein the therapy comprises the administration of a VWF concentrate.

## Patentansprüche

1. In-vitro-Diagnoseverfahren zur Bestimmung von qualitativen und/oder quantitativen Defekten des von-Willebrand-Faktors, **gekennzeichnet durch**,
a) Verwenden einer aus einem menschlichen Individuum oder einem Tier erhaltenen Probe, die eine Körperflüssigkeit umfasst, welche Blutplättchen und Hämostase-Faktoren umfasst,
b) Inkubieren mindestens eines Teils der Probe mit einem Aktivator der Blutplättchenenaggregation, wobei der Aktivator der Blutplättchenenaggregation eine Verbindung ist, die die VWF-abhängige Blutplättchenaktivierung, Aggregation und Agglutination in einer aus dem menschlichen Körper stammenden Flüssigkeit, die Blutplättchen und VWF enthält, induzieren kann, indem die Bindung von VWF an einen VWF-Rezeptor auf den Blutplättchen herbeigeführt wird,
c) Induzieren der Gerinnung in dem Teil der Probe, der mit einem Aktivator der Blutplättchenaggregation inkubiert wurde,
d) Messen der viskoelastischen Änderung, die nach der Induktion der Gerinnung in dem Teil der Probe erfolgt, der mit einem Aktivator der Blutplättchenaggregation inkubiert wurde, in einem Thromboelastographen, und
e) Vergleichen des Ausmaßes der in Schritt (d) bestimmten viskoelastischen Änderung mit einem Referenzwert,
wobei der Referenzwert erhalten wird:
i) **durch** Aufteilen der in Schritt (a) erhaltenen ursprünglichen Spenderprobe in mindestens zwei Proben oder Gewinnen von mindestens zwei Proben aus einem Spender in Schritt (a), wobei an mindestens einer Probe nur Schritt (a) und die Schritte (c) bis (e) durchgeführt werden und der Schritt (b) weggelassen wird, und wobei das Ausmaß der viskoelastischen Änderung, die in der Probe gemessen wurde, bei der der Schritt (b) weggelassen wurde, der Referenzwert ist; oder
ii) **durch** Anwenden eines Verfahrens, das den Schritt (a) und die Schritte (c) bis (e) umfasst, bei einer Probe aus einem gesunden Spender oder bei mehreren Proben aus gesunden Spendern ohne erhöhtes Blutungsrisiko, wobei das Ausmaß der viskoelastischen Änderung, die in der Probe oder in den Proben gemessen wurde, in der/denen Schritt (b) weggelassen wurde, der Referenzwert ist; oder
iii) **durch** Anwenden eines Verfahrens, das die Schritte (a) bis (e) umfasst, bei einer Probe aus einem gesunden Spender oder bei mehreren Proben aus gesunden Spendern ohne erhöhtes Blutungsrisiko, wobei das Ausmaß der in der Probe oder in den Proben gemessenen viskoelastischen Änderung der Referenzwert ist.

2. In-vitro-Diagnoseverfahren nach Anspruch 1, wobei die Körperflüssigkeit Vollblut, antikoaguliertes Blut oder blutplättchenreiches Plasma ist.

3. In-vitro-Diagnoseverfahren nach den Ansprüchen 1 oder 2, wobei der in Alternative (ii) oder (iii) von Anspruch 1 erhaltene Referenzwert ein Mittelwert der Ausmaße der viskoelastischen Änderung ist, wie sie in mehreren aus gesunden Spendern erhaltenen Proben gemessen wird.

4. In-vitro-Diagnoseverfahren nach den Ansprüchen 1 bis 3, wobei ein Gerinnungsaktivator zugegeben wird.

5. In-vitro-Diagnoseverfahren nach den Ansprüchen 1 bis 4, wobei ein Gerinnungsaktivator vor Schritt (b) oder nach Schritt (b), aber vor der Induktion der Gerinnung in Schritt (c) hinzugefügt wird .

6. In-vitro-Diagnoseverfahren nach den Ansprüchen 1 bis 5, wobei die viskoelastische Änderung durch Bestimmen von irgendeinem der Thromboelastographie-Parameter gemessen wird.

7. In-vitro-Diagnoseverfahren nach Anspruch 6, wobei der Thromboelastographie-Parameter die Maximal-Amplitude ist.

8. In-vitro-Diagnoseverfahren nach den Ansprüchen 1 bis 7, wobei der Aktivator der Blutplättchenaggregation aus der Gruppe ausgewählt ist, die Ristocetin und Botrocetin umfasst.

9. In-vitro-Diagnoseverfahren nach Anspruch 8, wobei der Aktivator der Blutplättchenaggregation Ristocetin ist.

10. In-vitro-Diagnoseverfahren nach Anspruch 9, wobei die Ristocetin-Konzentration zwischen 0,1 mg/ml und 1,5 mg/ml liegt.

11. In-vitro-Diagnoseverfahren nach den Ansprüchen 1 bis 10 zum Prognostizieren des Blutungsrisikos eines Patienten vor einer Operation oder während einer Therapie.

12. In-vitro-Diagnoseverfahren nach den Ansprüchen 1 bis 11, wobei die Körperflüssigkeitsprobe höchstens 105 µl pro Test ausmacht.

13. In-vitro-Diagnoseverfahren nach den Ansprüchen 1 bis 12, wobei ein Wert von (viskoelastische Änderung in einer Probe einer Körperflüssigkeit aus einem Spender mit zugesetztem Aktivator der Blutplättchenenaggregation/(viskoelastische Änderung in einer Probe der gleichen Körperflüssigkeit ohne zugesetzten Aktivator der Blutplättchenaktivierung) größer oder gleich 0,15 als Indikator für ein Blutungsrisiko interpretiert wird.

14. Verwendung des In-vitro-Diagnoseverfahrens nach den Ansprüchen 1 bis 13 zur Therapiekontrolle, wobei die Therapie die Verabreichung von DDAVP umfasst

15. Verwendung des In-vitro-Diagnoseverfahrens nach den Ansprüchen 1 bis 13 zur Therapiekontrolle, wobei die Therapie die Verabreichung eines VWF-Konzentrats umfasst.

## Revendications

1. Méthode de diagnostic *in vitro* pour déterminer des défauts qualitatifs et/ou quantitatifs du facteur de von Willebrand, **caractérisée par**,
a) l'utilisation d'un échantillon obtenu à partir d'un individu humain ou d'un animal comprenant un fluide corporel qui contient des plaquettes et des facteurs hémostatiques,
b) l'incubation d'au moins une partie de l'échantillon avec un activateur d'agrégation plaquettaire, où l'activateur d'agrégation plaquettaire est un composé pouvant induire une activation des plaquettes dépendante du facteur VWF, une agrégation et une agglutination dans un fluide dérivé de l'organisme humain qui contient des plaquettes et VWF en provoquant une liaison de VWF à un récepteur de VWF sur les plaquettes,
c) l'induction d'une coagulation dans la partie de l'échantillon qui a été incubée avec un activateur d'agrégation plaquettaire,
d) la mesure des variations viscoélastiques qui ont lieu après induction de la coagulation dans la partie de l'échantillon qui a été incubée avec un activateur d'agrégation plaquettaire dans un thromboélastographe, et
e) la comparaison de la quantité de la variation viscoélastique déterminée dans l'étape (d) avec une valeur de référence,
où la valeur de référence est obtenue :
i) en divisant l'échantillon de donneur initial obtenu dans l'étape (a) en au moins deux échantillons ou en obtenant dans l'étape (a) au moins deux échantillons chez un donneur où pour au moins l'un des échantillons, seule l'étape (a) et les étapes (c) à (e) sont mises en oeuvre et l'étape (b) est ignorée, et où la quantité de variation viscoélastique mesurée dans l'échantillon dans lequel l'étape (b) est omise est la valeur de référence ; ou
ii) en appliquant une méthode comprenant l'étape (a) et les étapes (c) à (e) à un échantillon provenant d'un donneur sain ou à plusieurs échantillons provenant de donneurs sains sans risque hémorragique augmenté, où la quantité de variation viscoélastique mesurée dans l'échantillon ou dans les échantillons dans lesquels l'étape (b) est omise est la valeur de référence ; ou
iii) en appliquant une méthode comprenant les étapes (a) à (e) à un échantillon provenant d'un donneur sain ou à plusieurs échantillons provenant de donneurs sains sans risque hémorragique augmenté, où la quantité de variation viscoélastique mesurée dans l'échantillon ou dans les échantillons est la valeur de référence.

2. Méthode de diagnostic *in vitro* selon la revendication 1, où le fluide corporel est le sang total, le sang anticoagulé ou le plasma riche en plaquettes.

3. Méthode de diagnostic *in vitro* selon les revendications 1 ou 2, où la valeur de référence obtenue dans l'alternative (ii) ou (iii) selon la revendication 1 est une valeur moyenne des quantités de variation viscoélastique mesurées dans plusieurs échantillons provenant de donneurs sains.

4. Méthode de diagnostic *in vitro* selon les revendications 1 à 3, où un activateur de coagulation est ajouté.

5. Méthode de diagnostic *in vitro* selon les revendications 1 à 4, où un activateur de régulation est ajouté avant l'étape (b) ou après l'étape (b) mais avant d'induire une coagulation dans l'étape (c).

6. Méthode de diagnostic *in vitro* selon les revendications 1 à 5, où la variation viscoélastique est mesurée en déterminant l'un quelconque des paramètres de thromboélastographie.

7. Méthode de diagnostic *in vitro* selon la revendication 6, où le paramètre de thromboélastographie est l'amplitude maximale.

8. Méthode de diagnostic *in vitro* selon les revendications 1 à 7, où l'activateur d'agrégation plaquettaire est choisi dans le groupe comprenant la ristocétine et la botrocétine.

9. Méthode de diagnostic *in vitro* selon la revendication 8, où l'activateur d'agrégation plaquettaire est la ristocétine.

10. Méthode de diagnostic *in vitro* selon la revendication 9, où la concentration en ristocétine est comprise entre 0,1 mg/ml et 1,5 mg/ml.

11. Méthode de diagnostic *in vitro* selon les revendications 1 à 10 destinée à la prédiction du risque hémorragique chez un patient avant opération chirurgicale ou pendant une thérapie.

12. Méthode de diagnostic *in vitro* selon les revendications 1 à 11, où l'échantillon de fluide corporel s'élève au maximum à 105 µl par test.

13. Méthode de diagnostic *in vitro* selon les revendications 1 à 12, où une valeur de (variation viscoélastique dans un échantillon d'un fluide corporel provenant d'un donneur avec ajout d'activateur d'agrégation plaquettaire)/(variation viscoélastique dans un échantillon du même fluide corporel sans ajout d'activateur d'activation plaquettaire) supérieure ou égale à 0,15 est interprétée comme étant un indicateur de risque hémorragique.

14. Utilisation de la méthode de diagnostic *in vitro* selon les revendications 1 à 13 pour contrôle d'une thérapie, où la thérapie comprend l'administration de DDAVP.

15. Utilisation de la méthode de diagnostic *in vitro* selon les revendications 1 à 13 pour contrôle d'une thérapie, où la thérapie comprend l'administration d'un concentré de VWF.
